# EUROPEAN PATENT SPECIFICATION

(11) **EP 0 927 146 B1**
(45) Date of publication and mention of the grant of the patent: **20.11.2002**
(21) Application number: 97909247.5
(22) Date of filing: 09.09.1997
(51) Int. Cl.: C07C 1/04, B01J 23/74, B01J 21/06

(54) **FISCHER-TROPSCH CATALYST AND PROCESS FOR PREPARING HYDROCARBONS**
FISCHER-TROPSCH KATALYSATOR UND VERFAHREN ZUR HERSTELLUNG VON KOHLENWASSERSTOFFEN
CATALYSEUR FISCHER-TROPSCH ET PROCEDE DE PREPARATION DES HYDROCARBURES

(30) Priority: 10.09.1996 EP 96202524
(43) Date of publication of application: 07.07.1999
(73) Proprietor: SHELL INTERNATIONALE RESEARCH MAATSCHAPPIJ B.V., 2596 HR Den Haag (NL)
(72) Inventor: BLANKENSTEIN, Paul, NL-1031 CM Amsterdam (NL); GEERLINGS, Jacobus, Johannes, Cornelis, NL-1031 CM Amsterdam (NL); HUISMAN, Hans, Michiel, NL-1031 CM Amsterdam (NL); LEDNOR, Peter, William, NL-1031 CM Amsterdam (NL); VAN LIEMT, Bernardus, Josephus, NL-1031 CM Amsterdam (NL)
(86) International application number: EP9704962
(87) International publication number: WO98011037

(56) References cited:
- US-A- 4 558 030
- US-A- 4 595 703
- US-A- 4 847 231
- US-A- 5 397 806

## Description

The present invention relates to a Fischer-Tropsch catalyst, the preparation of the said catalyst, and a process for the preparation of hydrocarbons from synthesis gas, that is a mixture of carbon monoxide and hydrogen.

The preparation of hydrocarbons from synthesis gas is well known in the art and is commonly referred to as Fischer-Tropsch synthesis.

Catalysts suitable for use in a Fischer-Tropsch synthesis process, that is Fischer-Tropsch catalysts, typically contain a catalytically active metal of Group VIII of the Periodic Table of the Elements (Handbook of Chemistry and Physics, 68th edition, CRC Press, 1987-1988). In particular, iron, nickel, cobalt and ruthenium are well known catalytically active metals for such catalyst. Cobalt has been found to be most suitable for catalyzing a process in which synthesis gas is converted to primarily paraffinic hydrocarbons containing 5 or more carbon atoms. In other words, the C₅⁺ selectivity of the catalyst is high.

Much research effort has been directed to finding alternative catalysts, preferably having a higher C₅⁺ selectivity than known catalysts at the same or higher activity.

European patent specification No. 178 008 discloses cobalt catalysts supported on a porous carrier, wherein most cobalt is concentrated in the peel of the catalyst particle.

European patent specification No. 167 215 discloses a cobalt/zirconia on silica catalyst for use in a fixed catalyst bed which catalyst satisfies a relation between the internal surface area and the external surface area.

European patent specification No. 363 537 describes an increase in activity of cobalt catalysts supported on titania, by adding up to 15% by weight of silica to the titania carrier, which is preferably rutile-rich.

European patent specification No. 370 757 describes a Fischer-Tropsch catalyst comprising a, preferably rutile-rich, titania support characterised in that there is incorporated with said titania support from 0.1 to 20 percent by weight of an inorganic binder selected from silica, alumina and zirconia.

European patent application publication No. 542 527 describes a Fischer-Tropsch catalyst comprising cobalt supported on a polymorph of anatase. The polymorph is of the formula Ti₁₋ₓMₓO₂ where x ranges from .01 to .14 and M is selected from the group consisting of silicon, zirconium, and tantalum.

European patent application publication No. 498 976 describes catalysts containing cobalt and rhenium supported on a (preferably rutile-rich) titania carrier. It is claimed that these catalysts have a high volumetric productivity (activity).

European patent specification No. 216 967 claims a catalyst composition useful for the conversion of methanol or synthesis gas to hydrocarbons comprising either cobalt or cobalt and thoria in catalytically active amount composited with titania or a titania-containing support, characterised in that the titania support is one having a rutile:anatase ratio of at least about 2:3. Catalyst compositions particularly preferred for use in a Fischer-Tropsch synthesis process are characterised in that the titania support has a rutile:anatase ratio in the range of from 4:1 to 100:1 or greater.

US-A-4 595 703 relates to Co/Ti catalysts comprising titania having a rutile : anatase ratio of at least 2:3.

Despite the research effort in this field there is still room for improvement. Accordingly, it would for example be desirable if an alternative catalyst could be found. It would be even more desirable if a catalyst could be found which has an even higher C₅⁺ selectivity at the same or, preferably, higher activity than known catalysts.

Thus, the present invention seeks to provide a catalyst and a process for the preparation of hydrocarbons which has advantages in one or more aspects with respect to prior art catalysts and processes.

In particular, most surprisingly it has now been found that certain disadvantages of anatase-rich carriers can be overcome by carefully controlling the surface area of the said carriers.

Therefore, according to an aspect of the invention, there is provided a Fischer-Tropsch catalyst comprising a compound of cobalt and/or a compound of iron supported on a titania catalyst carrier, and at least one promoter selected from Group IIIb, IVb, Vb, VIIb and/or VIII of the Periodic Table of the Elements, the said titania catalyst carrier having a rutile:anatase ratio of more than 1:100 and less than 2:3, and a surface area of less than 75 m²/g.

The rutile:anatase ratio is suitably determined by X-ray diffraction according to ASTM D 3720-78.

The surface area is suitably determined by the N₂ adsorption method described in ASTM D 4567-86.

The surface area of the catalyst carrier is preferably less than 65 m²/g, more preferably less than 55 m²/g. Without wishing to be bound to a particular theory, it would appear that catalytically inactive compounds of the catalytically active metal and titania, such as cobalt titanate, readily forms on anatase-rich carriers having a high surface area, like anatase-rich Fischer-Tropsch catalysts described in comparative experiments in European patent specification No. 216 967.

The surface area of the catalyst carrier is preferably more than 15 m²/g, preferably more than 25 m²/g, more preferably more than 35 m²/g.

Preferably, the titania catalyst carrier has a rutile:anatase ratio of less than 3:5, more preferably less than 1:2, even more preferably less than 1:3.

The rutile:anatase ratio is typically more than 1:100, preferably, more than 1:50, more preferably, more than 1:15.

According to a preferred embodiment, the Fischer-Tropsch catalyst carrier has a pore volume of at least 0.45 ml/g, preferably at least 0.50 ml/g, in particular at least 0.55 ml/g.

It has surprisingly been found that catalysts prepared from carriers having a pore volume of at least 0.45 ml/g are more active and selective than catalysts prepared from carriers having a lower pore volume.

The pore volume is suitably determined by Hg porosimetry according to ASTM D 4284-92 up to a pressure of 60000 psi (4.1 kbar).

The pore volume is typically less than 0.85 ml/g, in particular less than 0.75 ml/g. Higher pore volumes are in principle possible, but this may be detrimental to the strength of the catalyst carrier. This may be solved by adding binders such as silica, alumina or zirconia in an amount from 0.1 to 20 % by weight of the carrier. However, binders may have a negative influence on the performance of the catalyst. For example, the deactivation rate may be higher. Therefore, preferably, no binder is added.

The pore volume of the catalyst is preferably at least 0.20 ml/g, more preferably at least 0.25 ml/g, most preferably at least 0.30 ml/g. The pore volume is typically less than 0.80 ml/g, in particular less than 0.70 ml/g.

Catalytically active metals for selectively catalyzing the preparation of C₅⁺ hydrocarbons from synthesis gas are cobalt and/or iron, preferably cobalt.

It will be appreciated that the preferred amount of catalytically active metal present in the catalyst may vary, depending on the catalytically active metal that is used. Generally, however, the amount of catalytically active metal present in the catalyst may range from 0.1 to 100 parts by weight of metal per 100 parts by weight of carrier. In case cobalt or iron is applied the preferred amount may be in the range from 3 to 60 parts by weight, more preferably, from 5 to 50 parts by weight.

For Fischer-Tropsch catalysts according to the invention containing cobalt, it is advantageous to apply cobalt in an amount of at least 0.2 g cobalt/ml catalyst, more preferably at least 0.25 g cobalt/ml catalyst.

The amount of cobalt is preferably less than 0.8 g cobalt/ml catalyst, more preferably less than 0.6 g/ml, even more preferably less than 0.5 g/ml.

The above amount of cobalt refers to the total amount of cobalt, on the basis of cobalt metal, and can be determined by known elemental analysis techniques.

The catalyst further comprises one or more promoters selected from Group IIIb, IVb, Vb, VIIb and/or VIII of the Periodic Table of the Elements. Preferred promoters are selected from zirconium, manganese, scandium, vanadium, rhenium, platinum and palladium.

A most suitable catalyst comprises cobalt as the catalytically active metal and zirconium as a promoter. Another most suitable catalyst comprises cobalt as the catalytically active metal and manganese and/or vanadium as a promoter. The promoter may be incorporated in the catalyst using any of the methods discussed herein after with respect to the catalytically active component.

The promoter, if present in the catalyst, is typically present in an amount of from 0.1 to 60 parts by weight, preferably from 0.5 to 40 parts by weight, per 100 parts by weight of carrier material. It will however be appreciated that the optimum amount of promoter may vary for the respective elements which act as promoter.

It has surprisingly been found that a catalyst comprising as catalytically active metal cobalt and a small amount of manganese and/or vanadium, exhibits a higher C₅⁺ selectivity and a higher activity when used in a process for the preparation of hydrocarbons, compared to catalysts which are otherwise the same but containing cobalt only.

Therefore, according to a preferred embodiment the present invention relates to a catalyst comprising cobalt and manganese and/or vanadium, wherein the cobalt:(manganese + vanadium) atomic ratio is at least 12:1.

Typically, the cobalt-containing catalysts according to a preferred embodiment of the present invention do not contain alkali or alkaline earth metals, apart from possible impurities introduced with starting materials in the preparation process of the catalysts. Typically, the molar ratio of alkali or alkaline earth metals to cobalt metal is less than 0.01, preferably, less than 0.005.

Preferably, the cobalt:(manganese + vanadium) atomic ratio is at most 1500:1; more preferably at most 500:1; still more preferably at most 100:1, most preferably at most 38:1. The cobalt:(manganese + vanadium) atomic ratio is preferably at least 15:1; more preferably at least 16:1; still more preferably at least 18:1.

In addition to manganese and/or vanadium, the catalyst may comprise one or more additional promoters selected from Group IVB, the noble metals of Group VIII of the Periodic Table or rhenium, scandium, niobium or tantalum. Preferred additional promoters include zirconium, titanium, ruthenium, platinum, palladium and/or rhenium. The amount of additional promoter, if present, is typically between 1 and 150 parts by weight per 100 parts by weight of refractory oxide carrier.

The catalyst according to the present invention may suitably be prepared by a) mixing a powder of a titania having a rutile:anatase ratio of less than 2:3, or a titania precursor capable of transforming into a titania having the said rutile:anatase ratio, with a liquid; b) evaporating the liquid from the mixture to obtain solid, agglomerated particles; optionally followed by c) a crushing step, and incorporating a compound of cobalt and/or a compound of iron in the catalyst.

Preferably, the catalyst is further subjected to a calcination treatment. The calcination treatment may be carried out after step b) and/or step c), and/or after at least one catalytically active metal compound has been incorporated in the catalyst.

Typically, the rutile:anatase ratio of the titania powder or the anticipated rutile:anatase ratio of the titania precursor when transformed into titania, is equal to or smaller than the rutile:anatase ratio in the desired end product, that is the Fischer-Tropsch catalyst according to the present invention.

It has now been found that with this method, catalysts can be manufactured which have a higher pore volume, at a relatively low surface area, than catalysts obtained by other methods.

According to step a) of the process, a powder of a titania or a titania precursor is mixed with a liquid. The titania precursor is typically a precipitate of a titanium compound which can be transferred into titania upon calcination in air. Examples of suitable titania precursors are hydrated titania and titanium hydroxide.

An example of a suitable method to form a titania precursor precipitate is by precipitation from a solution of a titanium alkoxide. Examples of suitable alkoxides are titanium ethoxide, titanium n-propoxide, titanium isopropoxide, titanium n-butoxide, titanium isobutoxide and titanium tert-butoxide.

Titania powder is preferably used in step a) of the above process. The titania powder may be prepared by methods known in the art, such as by precipitation from a solution containing a titanium compound, to prepare a titania precursor precipitate, e.g. titanium hydroxide; followed by calcination to prepare titania. Examples of soluble titanium compounds are titanium alkoxides, titanium chlorides and other inorganic or organic titanium salts. It has however been found that traces of sulphur may have a detrimental effect on the performance of the catalysts. Therefore, it is preferred to apply titania powder which has not been prepared by a method which uses sulphur-containing compounds in the process.

A preferred titania powder is one which has been prepared pyrogenically by high temperature hydrolysis of a vaporisable titanium compound, such as titanium tetrachloride. A suitable method has been disclosed in German patent specification No. 870 242. The titania obtainable by this method is hereinafter referred to as fumed titania. Thus, in a preferred aspect, fumed titania is applied in the preparation of the titania carrier to be used in the catalysts according to the present invention.

The powder of titania or titania precursor is typically substantially insoluble in the liquid used in step a) . The liquid is suitably water and/or an organic compound. Suitable organic compounds are those which leave no organic traces like coke in the catalyst carrier following drying and/or calcination, and are substantially inert in admixture with the powder of titania and/or a titania precursor.

Typically, the mixture comprises from 20 to 95% by volume of liquid, preferably from 50 to 95% by volume.

According to step b) of the process, the liquid is evaporated from the mixture to obtain solid, agglomerated particles. This step can typically be effected by a known drying method such as spray-drying, flash-drying or oven-drying. An advantage of spray-drying is that if the desired particle size is within the range from 3 to 300 µm, the desired particle size can be manufactured directly, without the need for a crushing step. Other drying methods generally produce larger particles. These methods, however, may also be desired for the preparation of catalyst particles in the range from 3 to 300 µm, for example if it is desired to apply catalytically active metals onto the particles by an impregnation method as will be set out in more detail hereinafter.

It will be appreciated by those skilled in the art that the most preferred method of drying may vary, depending e.g. on the desired size of the catalyst particles. It belongs to the skill of the skilled person to select the most suitable method and the most suitable conditions for a given set of circumstances and requirements.

Optionally, according to step c) of the process, the particles obtained in step b) of the process are crushed to a desired average particle size. According to another embodiment, it is also possible to crush the particles obtained in step b), after the particles have been subjected to an additional calcining treatment.

The catalytically active metal may be applied to the carrier by any of the techniques known to those skilled in the art, for example impregnation, spray-coating or precipitation.

A preferred method of preparing the catalyst according to the present invention is by impregnating the catalytically active metal or precursor onto the titania carrier. Typically, the carrier is contacted with a compound of the catalytically active metal in the presence of a liquid, most conveniently in the form of a solution of the metal compound. The compound of the active metal may be inorganic or organic, with inorganic compounds being preferred, in particular nitrates. The liquid employed may also be either organic or inorganic. Water is a most convenient liquid. It will be appreciated that the water may, at least partly, be derived from crystal water which is liberated from the metal compound upon impregnation at elevated temperature.

The impregnation treatment is typically followed by drying. Drying is typically carried out at a temperature of 50 to 300 °C for up to 24 hours, preferably, 0.2 to 4 hours.

According to one embodiment, the process involves a crushing step c). It may be desired to impregnate the carrier prior to the crushing step as solids handling and impregnation of relatively large particles is generally easier.

If it is preferred to prepare catalysts containing an outer rim of catalytically active metal, for example as disclosed in European patent specification No. 178008, the impregnation step is carried out after the crushing step. Alternatively, a spray-coating method is applied after the crushing step to prepare such catalysts.

According to another preferred embodiment, one or more compounds of the one or more catalytically active metals are mixed with the powder of titania or titania-precursor in step a) of the process.

As set out above, preferably the catalyst is further subjected to a calcination treatment. The calcination treatment may be carried out after step b) and/or step c), and/or after at least one catalytically active metal compound has been incorporated in the catalyst.

The calcination treatment is typically carried out in an oxygen-containing atmosphere such as air at a temperature from 200 to 800 °C, for 0.2 to 48 hours. The higher the temperature and the longer the duration of the calcination treatment, the lower the pore volume, the lower the surface area, and the higher the rutile content in the titania particles. Most suitable calcination conditions can be determined by the skilled person by routine experimentation. Preferably, the calcination treatment is effected at a temperature in the range from 350 to 650 °C. The duration of the treatment is preferably from 0.5 to 4 hours.

It will be appreciated that the average temperature during the calcination treatment will normally be higher than the average temperature during the drying treatment of step b). Further, it will be appreciated that the drying and calcining step may be carried out as a one-step process.

According to one preferred embodiment, the calcination treatment is carried out after step b) or c), the calcined product is impregnated with solutions or molten salts of catalytically active compounds, and the impregnated product is subjected to a second calcination treatment. In that case it is generally preferred that the calcination conditions of the second calcination step are no more severe than the calcination conditions applied in the first calcination step.

The catalyst according to the present invention is typically used to catalyse a process for the preparation of hydrocarbons from synthesis gas. Typically, when in use in that process, at least part of the cobalt, typically at least 80 % by weight, is present in its metallic state. Preferably at least 90 % by weight of the cobalt is in the metallic state. Following the preparation process, however, most of the cobalt in the catalyst will not be in the metallic state. Therefore, it is normally advantageous to activate the catalyst prior to use by a reduction treatment, in the presence of hydrogen at elevated temperature. Typically, the reduction treatment involves treating the catalyst at a temperature in the range from 100 to 450 °C for 1 to 48 hours at elevated pressure, typically from 1 to 50 bar abs. Pure hydrogen may be used in the reduction treatment, but it is preferred to apply a mixture of hydrogen and an inert gas, like nitrogen. The relative amount of hydrogen present in the mixture may range between 0 and 100% by volume. In a preferred embodiment, the catalyst is brought to the desired temperature and pressure level in a nitrogen gas atmosphere. Subsequently, the catalyst is contacted with a gas mixture containing only a small amount of hydrogen gas, the rest being nitrogen gas. During the reduction treatment, the relative amount of hydrogen gas in the gas mixture is gradually increased up to 50% or even 100% by volume.

The quantity of the metal compound that has been reduced, can suitably be monitored by measurement of cumulative water production during the process, especially if most of the cobalt in the catalyst is in an oxidised state following a calcination treatment. Other methods known to those skilled in the art include Thermogravimetric Analysis and Temperature Programmed Reduction.

The reduction treatment is suitably controlled by means known in the art. Typically, the reduction treatment is controlled by preventing the water concentration in the off-gas to exceed a certain limit, which may depend on the catalyst that is reduced and can be determined by routine experimentation.

If possible, it is preferred to activate the catalyst in-situ, that is inside the reactor. In particular, if the reactor to be used in the process in a slurry bubble column or an ebulating bed reactor, it may be desirable to activate the catalyst in part or fully in the presence of liquid hydrocarbons. A method to partly reduce the catalyst in the presence of hydrocarbons has been disclosed in European patent application publication No. 0 589 692. Preferably, however, the catalyst is fully reduced in the presence of hydrocarbons. A most suitable method has been disclosed in co-pending European patent application No. 95203040.1, which method involves contacting the catalyst with a hydrogen-containing gas at a hydrogen partial pressure of at least 15 bar abs.

In a further aspect, the present invention relates to a process for the preparation of hydrocarbons, which comprises contacting a mixture of carbon monoxide and hydrogen at elevated temperature and pressure with a catalyst as described herein before.

The process is typically carried out at a temperature in the range from 125 to 350 °C, preferably 175 to 275 °C. The pressure is typically in the range from 5 to 150 bar abs., preferably from 5 to 80 bar abs.

Hydrogen and carbon monoxide (synthesis gas) is typically fed to the process at a molar ratio in the range from 0.4 to 2.5. It is known that especially low hydrogen to carbon monoxide molar ratios will increase the C₅⁺ selectivity of Fischer-Tropsch catalysts. However, it may be desirable to apply a hydrogen to carbon monoxide molar ratio of about 2, which corresponds to the ratio in which the synthesis gas is normally consumed, when using a catalyst having substantially no activity for CO-shift reactions.

It has now most surprisingly been found that the C₅⁺ selectivity of the catalyst according to the present invention is remarkably high, even when using synthesis gas having a high hydrogen to carbon monoxide molar ratio. Accordingly, in a preferred embodiment of the hydrocarbon synthesis process of the present invention, the hydrogen to carbon monoxide molar ratio is in the range from 1.0 to 2.5, more preferably in the range from 1.5 to 2.5.

The gas hourly space velocity may vary within wide ranges and is typically in the range from 400 to 10000 Nl/l/h (normal litre gas/litre catalyst particles/hour).

The process for the preparation of hydrocarbons may be conducted using a variety of reactor types and reaction regimes, for example a fixed bed regime, a slurry phase regime or an ebullating bed regime. It will be appreciated that the size of the catalyst particles may vary depending on the reaction regime they are intended for. It belongs to the skill of the skilled person to select the most appropriate catalyst particle size for a given reaction regime.

Further, it will be understood that the skilled person is capable to select the most appropriate conditions for a specific reactor configuration and reaction regime. For example, the preferred gas hourly space velocity may depend upon the type of reaction regime that is being applied. Thus, if it is desired to operate the hydrocarbon synthesis process with a fixed bed regime, preferably the gas hourly space velocity is chosen in the range from 500 to 2500 Nl/l/h. If it is desired to operate the hydrocarbon synthesis process with a slurry phase regime, preferably the gas hourly space velocity is chosen in the range from 1500 to 7500 Nl/l/h.

The invention will now be illustrated further by means of the following Examples.

### EXAMPLE I (comparative)

Commercially available titania particles (30-80 MESH) of the rutile variety (rutile:anatase 98:2) were impregnated with a concentrated solution containing cobalt nitrate and manganese nitrate.

The solution was prepared by heating solid cobalt nitrate (Co(NO₃)₂.6H₂O) and solid manganese nitrate (Mn(NO₃)₂.4H₂O) to a temperature of 60 °C, thus causing the metal nitrates to dissolve in their own crystal water. The impregnated titania particles were dried for 2 hours at 120 °C and subsequently calcined in air for 1 hours at 400 °C.

The catalyst (I) contained 10% by weight of metal compounds, expressed as metal. The cobalt:manganese atomic ratio amounted to 20:1. The porosity of the catalyst amounted to 30%, the pore volume of the catalyst was 0.1 ml/g, the pore volume of the titania carrier was 0.3 ml/g, and the surface area of the carrier was 13 m²/g. The cobalt loading amounted to 0.27 g/ml catalyst.

### EXAMPLE II

A spray-dried titania powder was prepared as follows. Commercially available fumed titania powder (P25 ex. Degussa) was mixed with water. The mixture contained 30% by weight of fumed titania powder. The mixture was spray-dried using a Niro Atomizer. The inlet temperature was 250 °C and the outlet temperature was 117 °C. The resulting product was calcined for 1 hour at 500 °C. The calcined product had an average particle diameter of 38 µm, and had a rutile:anatase ratio of 1:4.

The impregnation procedure of Example I was repeated but now the spray-dried titania powder was used as catalyst carrier.

The porosity of the catalyst (II) was 65%, the pore volume of the catalyst was 0.38 ml/g, the pore volume of the titania carrier was 0.6 ml/g, and the surface area of the carrier was 50 m²/g. The cobalt loading amounted to 0.29 g / ml catalyst.

### EXAMPLE III

Catalysts I and II were tested in a process for the preparation of hydrocarbons. Microflow reactors A and B, containing 3.5 ml of catalysts I and II respectively, were heated to a temperature of 260 °C, and pressurised with a continuous flow of nitrogen gas to a pressure of 2 bar abs. The catalysts were reduced in-situ for 24 hours with a mixture of nitrogen and hydrogen gas. During reduction, the relative amount of hydrogen in the mixture was gradually increased from 0% to 100%. The water concentration in the off-gas was kept below 3000 ppmv.

Following reduction, the pressure was increased to 26 bar abs. The reaction was carried out with a mixture of hydrogen and carbon monoxide at a H₂/CO molar ratio of 2:1. The GHSV amounted to 2400 Nl/l/h and the reaction temperature was 225 °C.

The space time yield (STY), expressed as grammes hydrocarbon product per litre catalyst particles (excluding voids between the particles) per hour, and the C₅⁺ selectivity, expressed as a weight percentage of the total hydrocarbon product, were determined for each of the reactors after 100 hours of operation.

The results are set out in Table I.

**TABLE I**

| Reactor: | A | B |
|---|---|---|
| Catalyst | I | II |
| STY (g/l/h) | 465 (499) | 540 |
| C₅⁺ select. (%) | 85 | 88 |

It will be appreciated that both the activity and the selectivity of catalyst II, according to the invention, is much better than the activity and selectivity of catalyst I, not according to the invention. This is also the case if the STY of catalyst I is corrected in view of the slightly lower cobalt loading per ml catalyst.

## Claims

1. A Fischer-Tropsch catalyst comprising a compound of cobalt and/or a compound of iron supported on a titania catalyst carrier, and at least one promoter selected from Group IIIb, IVb, Vb, VIIb and/or VIII of the Periodic Table of the Elements, the said titania catalyst carrier having a rutile:anatase ratio of more than 1:100 and less than 2:3, and a surface area of less than 75 m²/g.

2. A Fischer-Tropsch catalyst as claimed in claim 1, wherein the carrier has a surface area of less than 65 m²/g.

3. A Fischer-Tropsch catalyst as claimed in claim 1 or 2, wherein the titania catalyst carrier has a rutile:anatase ratio of less than 3:5, and wherein the carrier has a pore volume as determined by Hg porosimetry of at least 0.45 ml/g, preferably a pore volume of at least 0.50 ml/g.

4. A Fischer-Tropsch catalyst as claimed in any one of the preceding claims, comprising a cobalt compound.

5. A Fischer-Tropsch catalyst as claimed in claim 4, wherein cobalt is present in an amount of at least 0.2 g cobalt/ml catalyst, and preferably comprising at least one promotor selected from manganese and vanadium.

6. A Fischer-Tropsch catalyst as claimed in claim 5, wherein the cobalt:(manganese + vanadium) atomic ratio is at least 12:1.

7. A Fischer-Tropsch catalyst as claimed in any one of the preceding claims 1 to 6, wherein at least part of the cobalt and/or iron is in the metallic state, preferably wherein cobalt is present and at least 90% by weight of the cobalt is in the metallic state.

8. A process for the preparation of a Fischer-Tropsch catalyst as claimed in any one of the preceding claims 1 to 6 which comprises
a) mixing a powder of a titania having a rutile:anatase ratio of less than 2:3, or a titania precursor capable of transforming into a titania having the said rutile:anatase ratio, with a liquid;
b) evaporating the liquid from the mixture to obtain solid, agglomerated particles; optionally followed by
c) a crushing step; and
incorporating a compound of cobalt and/or a compound of iron in the catalyst.

9. A process for the preparation of a Fischer-Tropsch catalyst as claimed in claim 7, which comprises subjecting a catalyst as claimed in any one of claims 1 to 6 to a reduction treatment in the presence of hydrogen.

10. A process for the preparation of hydrocarbons, which comprises contacting a mixture of carbon monoxide and hydrogen at elevated temperature and pressure with a catalyst as claimed in claim 7, preferably at a temperature in the range from 125 to 350 °C and a pressure in the range from 5 to 150 bar abs.

## Patentansprüche

1. Fischer-Tropsch-Katalysator, umfassend eine Verbindung von Kobalt und/oder eine Verbindung von Eisen, aufgebracht auf einen Titanoxidkatalysatorträger, und wenigstens einen Promotor, ausgewählt aus Gruppe IIIb, IVb, Vb, VIIb und/oder VIII des Periodensystems der Elemente, wobei dieser Titanoxidkatalysatorträger ein Rutil:Anatas-Verhältnis von über 1:100 und unter 2:3 sowie eine Oberflächengröße von weniger als 75 m²/g aufweist.

2. Fischer-Tropsch-Katalysator nach Anspruch 1, worin der Träger eine Oberflächengröße von weniger als 65 m²/g aufweist.

3. Fischer-Tropsch-Katalysator nach Anspruch 1 oder 2, worin der Titanoxidkatalysatorträger ein Rutil:Anatas-Verhältnis von weniger als 3:5 aufweist und worin der Träger ein Porenvolumen, bestimmt durch Quecksilberporosimetrie, von wenigstens 0,45 ml/g, vorzugsweise ein Porenvolumen von wenigstens 0,50 ml/g hat.

4. Fischer-Tropsch-Katalysator nach einem der vorstehenden Ansprüche, umfassend eine Kobaltverbindung.

5. Fischer-Tropsch-Katalysator nach Anspruch 4, worin das Kobalt in einer Menge von wenigstens 0,2 g Kobalt/ml Katalysator vorliegt und vorzugsweise mit einem Gehalt an wenigstens einem Promotor, ausgewählt unter Mangan und Vanadium.

6. Fischer-Tropsch-Katalysator nach Anspruch 5, worin das Kobalt: (Mangan + Vanadium)-Atomverhältnis wenigstens 12:1 beträgt.

7. Fischer-Tropsch-Katalysator nach einem der vorstehenden Ansprüche 1 bis 6, worin wenigstens ein Teil des Kobalts und/oder Eisens in metallischem Zustand vorliegt, wobei vorzugsweise Kobalt zugegen ist und wenigstens 90 Gew.-% des Kobalts in metallischem Zustand vorliegen.

8. Verfahren zur Herstellung eines Fischer-Tropsch-Katalysators, wie in einem der vorstehenden Ansprüche 1 bis 6 beansprucht, das
a) ein Vermischen eines Pulvers eines Titanoxids mit einem Rutil:Anatas-Verhältnis von kleiner als 2:3 oder eines Titanoxidvorläufers, der zur Umwandlung in ein Titanoxid mit diesem Rutil:Anatas-Verhältnis befähigt ist, mit einer Flüssigkeit;
b) ein Verdampfen der Flüssigkeit aus dem Gemisch, um feste, agglomerierte Teilchen zu ergeben; gegebenenfalls gefolgt von
c) einer Zerkleinerungsstufe, und ein Einbringen einer Kobaltverbindung und/oder einer Eisenverbindung in den Katalysator umfaßt.

9. Verfahren zur Herstellung eines Fischer-Tropsch-Katalysators nach Anspruch 7, das die Ausführung einer Reduktionsbehandlung eines Katalysators, wie in einem der Ansprüche 1 bis 6 beansprucht, in Anwesenheit von Wasserstoff umfaßt.

10. Verfahren zur Herstellung von Kohlenwasserstoffen, das ein Inkontaktbringen eines Gemisches aus Kohlenmonoxid und Wasserstoff bei erhöhter Temperatur und erhöhtem Druck mit einem Katalysator, wie in Anspruch 7 beansprucht, vorzugsweise bei einer Temperatur im Bereich von 125 bis 350°C und bei einem Druck im Bereich von 5 bis 150 bar absolut, umfaßt.

## Revendications

1. Catalyseur de Fischer-Tropsch comprenant un composé de cobalt et/ou un composé de fer fixés sur un support de catalyseur d'oxyde de titane, et au moins un promoteur choisi parmi les Groupes IIIb, IVb, Vb, Vllb et/ou VIII du Tableau Périodique des Eléments, ledit support de catalyseur d'oxyde de titane ayant un rapport rutile/anatase supérieur à 1/100 et inférieur à 2/3, et une aire superficielle inférieure à 75 m²/g.

2. Catalyseur de Fischer-Tropsch suivant la revendication 1, dans lequel le support a une aire superficielle inférieure à 65 m²/g.

3. Catalyseur de Fischer-Tropsch suivant l'une ou l'autre des revendications 1 et 2, dans lequel le support de catalyseur d'oxyde de titane a un rapport rutile/anatase inférieur à 3/5, et dans lequel le support a un volume des pores tel que déterminé par une porosimétrie au Hg d'au moins 0,45 ml/g, avantageusement un volume des pores d'au moins 0,50 ml/g.

4. Catalyseur de Fischer-Tropsch suivant l'une quelconque des revendications précédentes, comprenant un composé de cobalt.

5. Catalyseur de Fischer-Tropsch suivant la revendication 4, dans lequel le cobalt est présent en une quantité d'au moins 0,2 g de cobalt/ml de catalyseur, et avantageusement comprenant au moins un promoteur choisi parmi le manganèse et le vanadium.

6. Catalyseur de Fischer-Tropsch suivant la revendication 5, dans lequel le rapport atomique cobalt/manganèse + vanadium) est d'au moins 12/1.

7. Catalyseur de Fischer-Tropsch suivant l'une quelconque des revendications 1 à 6, dans lequel au moins une partie du cobalt et/ou du fer est à l'état métallique, avantageusement dans lequel du cobalt est présent et au moins 90% en poids du cobalt sont à l'état métallique.

8. Procédé de préparation d'un catalyseur de Fischer-Tropsch suivant l'une quelconque des revendications 1 à 6, qui comprend :
a) le mélange d'une poudre d'un oxyde de titane ayant un rapport rutile/anatase inférieur à 2/3, ou d'un précurseur d'oxyde de titane pouvant se transformer en un oxyde de titane ayant ledit rapport rutile/anatase, avec un liquide ;
b) l'évaporation du liquide du mélange pour obtenir des particules agglomérées, solides, éventuellement suivie
c) d'une étape de broyage; et
l'incorporation d'un composé de cobalt et/ou d'un composé de fer dans le catalyseur.

9. Procédé de préparation d'un catalyseur de Fischer-Tropsch suivant la revendication 7, qui consiste à soumettre un catalyseur suivant l'une quelconque des revendications 1 à 6 à un traitement de réduction en présence d'hydrogène.

10. Procédé de préparation d'hydrocarbures, qui comprend la mise en contact d'un mélange de monoxyde de carbone et d'hydrogène à température et pression élevées avec un catalyseur suivant la revendication 7, avantageusement à une température allant de 125 à 350°C et une pression allant de 5 à 150 bars absolus.
